# EUROPEAN PATENT APPLICATION

(11) **EP 1 887 021 A1**
(43) Date of publication of application: **13.02.2008**
(21) Application number: 06746469.3
(22) Date of filing: 16.05.2006
(51) Int. Cl.: C08G 59/20, C07D 301/27

(54) **HARDENABLE COMPOSITION AND NOVEL ADAMANTANE COMPOUND**

(30) Priority: 17.05.2005 JP 2005144324
(71) Applicant: Asahi Glass Company, Limited, Chiyoda-ku, Tokyo 100-8405 (JP)
(72) Inventor: SUGIYAMA, Norihide Asahi Glass Company, Limited, Chiyoda-ku, Tokyo 1008405 (JP)
(74) Representative: Müller-Boré & Partner Patentanwälte
(86) International application number: PCT/JP2006/309755
(87) International publication number: WO 2006/123664

(57) **Abstract**

To provide a curable composition to form a cured fluorinated product excellent in light resistance, transparency, moisture resistance and adhesive properties, and a novel adamantane compound.

A liquid curable composition which comprises a fluoropolymer having a group capable of reacting with an epoxy group or an acid anhydride and containing polymerized units based on a fluoroolefin and polymerized units based on a hydrocarbon monomer, a fluorinated compound having an epoxy group, and an acid anhydride. An adamantane compound having from one to four of hydrogen atoms in the adamantine ring substituted by a glycidyl ether group and at least six of the remaining hydrogen atoms substituted by a fluorine atom.

## Description

### TECHNICAL FIELD

The present invention relates to a curable composition capable of easily forming a cured fluorinated product excellent in optical properties, etc., a cured fluorinated product formed by curing the curable composition, and an optical article having the cured fluorinated product. Further, it relates to a novel adamantane compound useful as one component of the curable composition.

### BACKGROUND ART

A white LED lamp having a white light emitting diode (hereinafter referred to as white LED) using a light emitting diode emitting short wavelength light (e.g. light at a wavelength of 460 nm, 405 nm or 380 nm) and a phosphor for wavelength conversion encapsulated with a transparent resin in a light-transmitting manner, has been used as e.g. a backlight of a mobile phone. In recent years, it has been actively attempted to use white lamp using a higher luminance white LED to various light equipment.

For such a transparent resin, physical properties such as light resistance, transparency, moisture resistance and adhesive properties are required. Further, from the viewpoint of production efficiency, the transparent resin is preferably formed directly from a liquid curable material and the like.

As a transparent resin, an alicyclic epoxy resin with small near ultraviolet absorption has been proposed (Patent Document 1). However, a transparent resin is likely to deteriorate by the synergistic effect of light and heat of LED, and light resistance and heat resistance of an alicyclic epoxy resin are insufficient yet. Further, as a transparent resin having high light resistance and heat resistance, a silicone resin has been proposed (Patent Document 2), but a silicone resin has insufficient moisture resistance and adhesive properties and has low hardness.

As a transparent resin excellent in light resistance and heat resistance, a thermoplastic amorphous fluororesin has been proposed (Patent Documents 3 and 4). However, such a resin is non-adhesive and has low adhesive properties.

Further, such a resin is thermoplastic, thus providing a low production efficiency in an encapsulation process since the resin must be melted and fluidized at high temperature (about 300°C) for encapsulation in a light-transmitting manner. Further, in Patent Document 4, a transparent resin is formed by distilling a solvent from a coating agent in the form of a solution containing the thermoplastic amorphous fluororesin and the solvent, and accordingly it is not easy to obtain a thickness (at' least 100 µm) required to encapsulate LED.
Patent Document 1: JP-A-11-274571
Patent Document 2: JP-A-2002-374007
Patent Document 3: JP-A-2001-102639
Patent Document 4: JP-A-2003-8073

### DISCLOSURE OF THE INVENTION

### OBJECT TO BE ACCOMPLISHED BY THE INVENTION

The object of the present invention is to provide a curable composition capable of forming a cured fluorinated product excellent in light resistance, transparency, moisture resistance and adhesive properties, and an optical article using it.

### MEANS TO ACCOMPLISH THE OBJECT

The present invention provides the following.
(1) A liquid curable composition which comprises a fluoropolymer having a group capable of reacting with an epoxy group or an acid anhydride and containing polymerized units based on a fluoroolefin and polymerized units based on a hydrocarbon monomer, a fluorinated compound having an epoxy group, and an acid anhydride.
(2) A cured fluorinated product, formed by curing the curable composition as defined in the above (1).
(3) An optical article having the cured fluorinated product as defined in the above (2).
(4) An adamantane compound having from one to four of hydrogen atoms in the adamantine ring substituted by a glycidyl ether group and at least six of the remaining hydrogen atoms substituted by a fluorine atom.
(5) The adamantane compound according to the above (4), which is represented by the following formula (a): 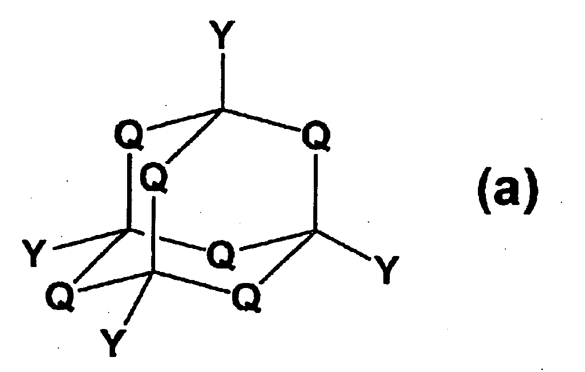 wherein symbols have the following meanings:
   Y: each independently a hydrogen atom, a fluorine atom or a glycidyl ether group, provided that one to four among four Y's is a glycidyl ether group, and
   Q: each independently -CHF- or -CF₂-.
(6) The adamantane compound according to the above (4) or (5), which is represented by the following formula (a1) : 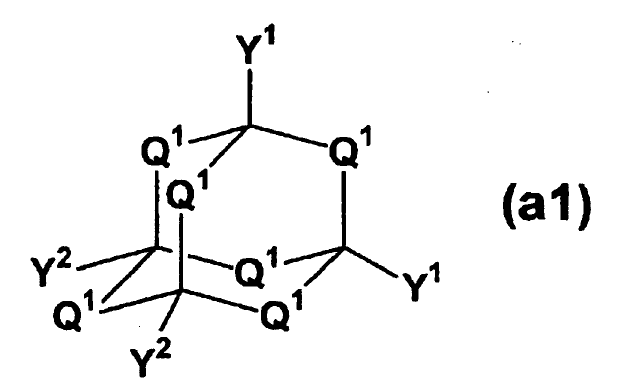 wherein symbols have the following meanings:
   Y¹: a glycidyl ether group,
   Y²: each independently a hydrogen atom or a fluorine atom, and
   Q¹: each independently -CHF- or -CF₂-.
(7) A cured fluorinated product formed by curing a liquid curable composition containing the adamantane compound as defined in any one of the above (4) to (6).
(8) A cured fluorinated product formed by curing a liquid curable composition containing the adamantane compound as defined in any one of the above (4) to (6) and an acid anhydride.
(9) A cured fluorinated product formed by curing a liquid curable composition containing the adamantane compound as defined in any one of the above (4) to (6), a fluoropolymer containing polymerized units based on a fluoroolefin and polymerized units based on a hydrocarbon monomer, and an acid anhydride.
(10) An optical article having the cured fluorinated product as defined in any one of the above (7) to (9).

### EFFECTS OF THE INVENTION

The curable composition of the present invention provides small volume shrinkage even when cured in an open system, and is capable of forming a cured fluorinated product having an optional shape. The cured fluorinated product of the present invention is excellent in transparency and light resistance to light at a wavelength of from 200 to 500 nm (hereinafter referred to as short wavelength light). Further, it is an insoluble and infusible crosslinked polymer and is excellent also in adhesive properties and heat resistance and is thereby suitable as a material for encapsulating an optical article in a light-transmitting manner. Further, according to the present invention, a novel reactive adamantane compound having a glycidyl ether group and fluorine atoms is provided. A cured fluorinated product excellent in the above properties is obtained also from the adamantane compound, and the product is suitable as a material for encapsulating an optical article in a light-transmitting manner.

### BEST MODE FOR CARRYING OUT THE INVENTION

In the present specification, for example, a compound represented by the formula (a) will be referred to as a compound (a), and a compound represented by the formula (a1) will be referred to as a compound (a1). The same applies to compounds represented by the other formulae.

The curable composition of the present invention is liquid. The viscosity of the curable composition is preferably from 0.1 to 200 Pa·S, particularly preferably from 1 to 100 Pa·S at 25°C considering convenience during use.

The curable composition of the present invention contains a fluoropolymer (hereinafter referred to as a curable fluoropolymer) having a group reactive with an epoxy group or an acid anhydride (hereinafter referred to as a reactive group) and containing polymerized units (hereinafter referred to as units A) based on a fluoroolefin and polymerized units (hereinafter referred to as units B) based on a hydrocarbon monomer. The curable composition of the present invention, which contains a curable fluoropolymer, provides small volume shrinkage when cured and is capable of forming a cured fluorinated product excellent in mechanical strength and light resistance.

The weight average molecular weight of the curable fluoropolymer is preferably from 1,000 to 100;000, particularly preferably from 3,000 to 20,000. Within this range, the curable fluoropolymer has a low viscosity, whereby the curable composition will easily be prepared. Further, a cured fluorinated product having high hardness will be obtained. The content of the curable fluoropolymer in the curable composition is preferably from 5 to 70 mass%, particularly preferably from 5 to 50 mass%, from the viewpoint of the viscosity of the curable composition.

The reactive group is preferably a group having active hydrogen or an acid anhydride group, more preferably a hydroxyl group, an amino group, a thiol group or an acid anhydride group, particularly preferably a hydroxyl group. In such a case, coloring of the curable composition by curing will be suppressed. The reactive group in the curable fluoropolymer is preferably a reactive group in the polymerized units based on a hydrocarbon monomer having a reactive group.

The units A are preferably polymerized units based on at least one fluoroolefin selected from the group consisting of vinyl fluoride, vinylidene fluoride, trifluoroethylene, chlorotrifluoroethylene, tetrafluoroethylene, pentafluoropropylene, hexafluoropropylene and perfluoro(propyl vinyl ether), more preferably polymerized units based on at least one fluoroolefin selected from the group consisting of vinylidene fluoride, chlorotrifluoroethylene, tetrafluoroethylene and hexafluoropropylene.

The content of the units A in the curable fluoropolymer is preferably from 10 to 90 mol%, particularly preferably from 30 to 80 mol%.

The units B preferably essentially contain polymerized units (hereinafter referred to as units B1) based on a hydrocarbon monomer having a reactive group.

The hydrocarbon monomer having a reactive group is preferably a hydroxyl group-containing alkyl alkenyl ether, a hydroxyl group-containing alkyl vinyl ester, a hydroxyl group-containing alkyl (meth)acrylate or a monomer containing an acid anhydride group. A (meth)acrylate includes an acrylate and a methacrylate. The molecular structure of the alkyl group may be any of a chain structure, a branched structure and a cyclic structure. The cyclic structure may be a monocyclic structure, a condensed cyclic structure, a spirocyclic structure or a crosslinked structure.

The hydroxyl group-containing alkyl alkenyl ether may, for example, be a hydroxyl group-containing alkyl vinyl ether such as ω-hydroxyethyl vinyl ether, ω-hydroxypropyl vinyl ether, ω-hydroxybutyl vinyl ether, cyclohexanedimethanol monovinyl ether, ω-hydroxybutyl isopropenyl ether or hydroxycyclohexyl vinyl ether; a hydroxyl group-containing alkyl allyl ether such as hydroxyethyl allyl ether, hydroxypropyl allyl ether, hydroxybutyl allyl ether, methyleneglycol monoallyl ether or propylene glycol monoallyl ether; or ω-hydroxybutyl isopropenyl ether.

Specifically, the hydroxyl group-containing alkyl vinyl ester may, for example, be vinyl hydroxyacetate, vinyl hydroxypropionate, vinyl hydroxybutyrate or vinyl hydroxycyclohexanecarboxylate.

Specifically, the hydroxy group-containing alkyl (meth)acrylate may, for example, be 2-hydroxyethyl (meth)acrylate or hydroxypropyl acrylate.

Specifically, the monomer containing an acid anhydride group may, for example, be maleic anhydride, itaconic anhydride or norbornene dicarboxylic anhydride.

The content of the units B1 in the curable fluoropolymer is preferably from 1 to 40 mol%, more preferably from 1 to 25 mol%.

The hydrocarbon monomer other than the hydrocarbon monomer having a reactive group may, for example, be an alkyl vinyl ether such as ethyl vinyl ether, propyl vinyl ether, (iso)butyl vinyl ether, 2-ethylhexyl vinyl ether or cyclohexyl vinyl ether; a hydrocarbon vinyl ester such as vinyl acetate, vinyl propionate, vinyl (iso)butyrate, vinyl valerate, vinyl cyclohexanecarboxylate or vinyl benzoate; an alkyl allyl ether such as ethyl allyl ether, propyl allyl ether, (iso)butyl allyl ether or cyclohexyl allyl ether; an olefin such as ethylene, propylene or isobutyrene; or an alkyl (meth)acrylate such as methyl (meth)acrylate, ethyl (meth)acrylate, propyl (meth)acrylate, (iso)butyl (meth)acrylate or 2-ethylhexyl (meth)acrylate.

The curable composition of the present invention further contains a fluorinated compound having an epoxy group (hereinafter referred to as a fluorinated epoxy compound). The content of the fluorinated epoxy compound in the curable composition is preferably from 20 to 70 mass%, particularly preferably from 40 to 70 mass% in view of the content of all fluorine atoms in the composition and the viscosity.

Specifically, the epoxy group may, for example, be an oxirane group, a glycidyl ether group or a group represented by the following formula (E):

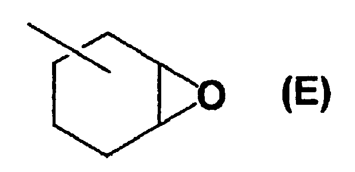

The fluorinated epoxy compound is preferably a fluorinated epoxy compound having from 2 to 6 epoxy groups, particularly preferably a fluorinated epoxy compound having from 2 to 4 epoxy groups, from the viewpoint of the curing density and the hardness of the cured fluorinated product. Further, a fluorinated epoxy compound having one epoxy group may be used so as to adjust the viscosity of the fluorinated curable composition.

The fluorinated epoxy compound may be either an aliphatic fluorinated epoxy compound or an aromatic fluorinated epoxy compound, preferably an aliphatic fluorinated epoxy compound, whereby a cured fluorinated product excellent in light resistance will be obtained.

Specific examples of the aliphatic fluorinated epoxy compound include an adamantane compound described hereinafter and the following compounds (x and y are each independently an integer of from 0 to 20, provided that the sum of x and y is from 1 to 20, and z is an integer of from 2 to 10) :

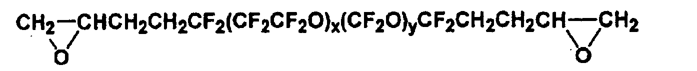

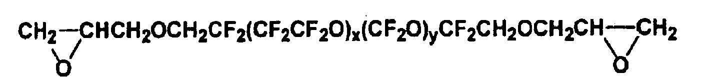

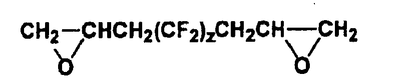

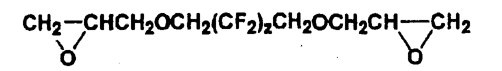

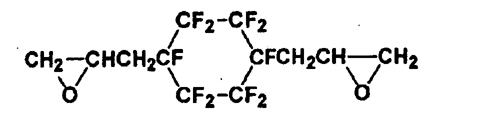

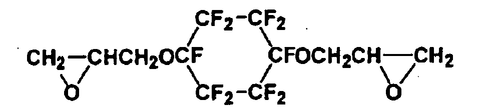

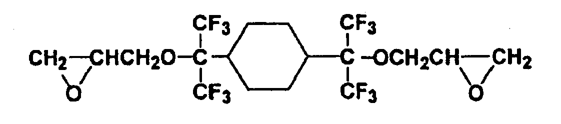

The curable composition of the present invention, which contains an acid anhydride, undergoes curing at an appropriate rate and is capable of forming a transparent and colorless cured fluorinated product. The content of the acid anhydride in the curable composition is preferably from 0.8 to 1.2 equivalent amounts, particularly preferably from 0.9 to 1.1 equivalent amounts per equivalent amount of the epoxy group in the fluorinated epoxy compound.

The acid anhydride may, for example, be glutaric anhydride, tetrahydrophthalic anhydride, methyltetrahydrophthalic anhydride, hexahydrophthalic anhydride, methylhexahydrophthalic anhydride or methyl nadic anhydride. The acid anhydride may contain a fluorine atom from the viewpoint of heat resistance and light resistance of the cured fluorinated product. The acid anhydride containing a fluorine atom may, for example, be perfluoroglutaric anhydride, perfluorosuccinic anhydride or tetrafluorophthalic anhydride.

The curable composition of the present invention may contain a component (hereinafter referred to as another component) other than the curable fluoropolymer, the fluorinated epoxy compound and the acid anhydride. Such another component may, for example, be a phosphor for LED wavelength conversion, an amine, a carboxylic acid, a curing accelerator, an antioxidant, or an inorganic filler such as silica fine particles for viscosity adjustment or for light distribution adjustment. The curable composition preferably contains a curing accelerator in order that curing is carried out under moderate conditions. The amount of the curing agent is preferably from 0.1 to 3 mass% based on the fluorinated epoxy compound.

The amine may, for example, be an aliphatic diamine or an aromatic diamine.

The carboxylic acid may, for example, be a dicarboxylic acid which may contain a fluorine atom (such as octafluoroadipic acid).

The curing accelerator may, for example, be an onium salt such as a tertiary amine, an imidazole compound, triphenylphosphine, a sulfonium salt or a phosphonium salt.

The curable composition of the present invention may contain a solvent when the respective components are mixed or the composition is prepared, but it preferably contains substantially no solvent when it is cured, from the viewpoint of the influence of the solvent at the time of heat curing and the solvent remaining in a cured fluorinated product obtained after curing over various physical properties (e.g. light resistance) of the cured fluorinated product. For example, the curable composition before cured may contain a solvent used to disperse or dissolve the curable fluoropolymer, the fluorinated epoxy compound and the acid anhydride. When it is cured, it is preferred to distill the solvent off to obtain a liquid curable composition containing substantially no solvent. In a case where the curable composition contains no solvent, such an effect will be obtained that the volume shrinkage of the curable composition by curing is small.

The curable composition of the preferably is cured by heat and/or by light irradiation. The temperature of the system during curing is preferably from 0 to 200°C, particularly preferably from 80°C to 150°C. Further, the temperature of the system may be changed so that the curing proceeds stepwise, with a view to relaxing a stress generated accompanying shrinkage on curing of the cured fluorinated product. Light irradiation for curing is preferably irradiation with ultraviolet rays, particularly preferably irradiation with light at a wavelength of from 250 to 500 nm. In such a case, the curable composition preferably contains a photocuring initiator as a curing accelerator. The curing time is not particularly limited and is preferably from 1 to 10 hours.

The curing may be carried out in a closed system (closed system) or may be carried out in a system not closed (open system). The curable composition of the present invention provides small volatilization of components and small volume shrinkage accompanying curing, and is thereby capable of being cured in an open system.

The cured fluorinated product of the present invention is an insoluble and infusible crosslinked polymer and is excellent in adhesive properties, and is excellent in light resistance to short wavelength light, transparency, heat resistance and moisture resistance. The cured fluorinated product of the present invention is useful as a material for an optical article, particularly a material for encapsulating an optical material in a light-transmitting manner. Therefore, the present invention also relates to an optical article having a cured fluorinated product. Encapsulation in a light-transmitting manner means encapsulation with both light-transmitting function and encapsulation function. The optical article is preferably a light emitting device or a light receiving device, particularly preferably a light emitting device. Specific examples of a light emitting device include a light emitting diode (LED), a laser light emitting diode (LD) and an electroluminescence (EL) device.

As a method for producing an optical article encapsulated with a cured fluorinated product obtained by curing the curable composition of the present invention in a light-transmitting manner, a method may be mentioned wherein a curable composition is applied to an optical article and then the curable composition is cured to form a cured fluorinated product. The method of applying the curable composition may be properly determined depending upon the shape of the optical article. It may, for example, a potting method by a dispenser, a screen printing method, a spin coating method or a die coating method.

The present invention further provides a novel adamantane compound containing a glycidyl ether group and fluorine atoms, useful as the above fluorinated epoxy compound or the like. In the present specification, an adamantane ring is a carbon ring composed of ten carbon atoms, which is a bridged tricyclic hydrocarbon compound represented by the following formula (Ad). The adamantane ring is composed of four tertiary carbon atoms and six secondary carbon atoms as represented by the formula (Ad).

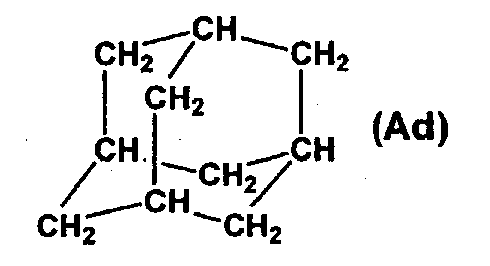

The adamantane compound of the present invention is a compound having from one to four of hydrogen atoms bonded to a carbon atom in the adamantane ring substituted by a glycidyl ether group and having six or more of the remaining hydrogen atoms substituted by a fluorine atom. An adamantane compound in which the remaining hydrogen atoms not substituted by a glycidyl ether group are not substituted by a fluorine atom, has a high melting point, and accordingly when it is mixed with an acid anhydride, a liquid curable composition may not be prepared, or the viscosity of the obtained curable composition may be very high. An adamantane compound in which many of the remaining hydrogen atoms not substituted by a glycidyl ether group are substituted by a fluorine atom, has a lowered melting point and is highly soluble in an acid anhydride, whereby a liquid curable composition having a low viscosity can be prepared. Further, a cured fluorinated product formed by curing such a curable composition is excellent in oxidation resistance.

From the above viewpoint, in the adamantane compound, it is preferred that all but one of the remaining hydrogen atoms not substituted by a glycidyl ether group are substituted by a fluorine atom, particularly preferably all such hydrogen atoms are substituted by a fluorine atom.

The adamantane compound of the present invention is preferably liquid or semisolid at room temperature (25°C). The melting point of the adamantane compound is preferably at most 100°C, more preferably at most 80°C, particularly preferably at most 60°C.

The adamantane compound of the present invention is preferably the following compound (a), particularly preferably the following compound (a1) .

Symbols in the formulae have the following meanings.

Y: Each independently a hydrogen atom, a fluorine atom or a glycidyl ether group, provided that one to four among four Y's is a glycidyl ether group.

Q: each independently -CHF- or -CF₂-,

Y¹: a glycidyl ether group,

Y²: each independently a hydrogen atom or a fluorine atom, and

Q¹: each independently -CHF- or -CF₂-.

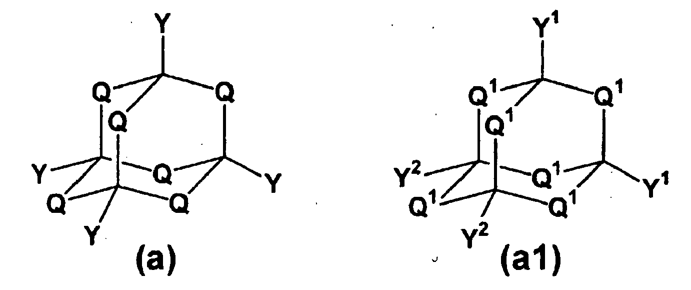

Y is each independently preferably a fluorine atom or a glycidyl ether. Further, it is more preferred that Y is each independently a fluorine atom or a glycidyl ether group, and Q is -CF₂-.

Y² is preferably a fluorine atom. Further, it is more preferred that Y² is a fluorine atom and Q¹ is -CF₂-.

Specific examples of the adamantane compound of the present invention include the following compounds:

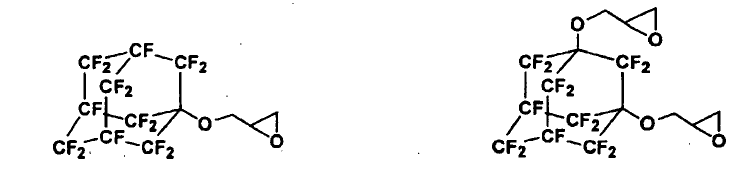

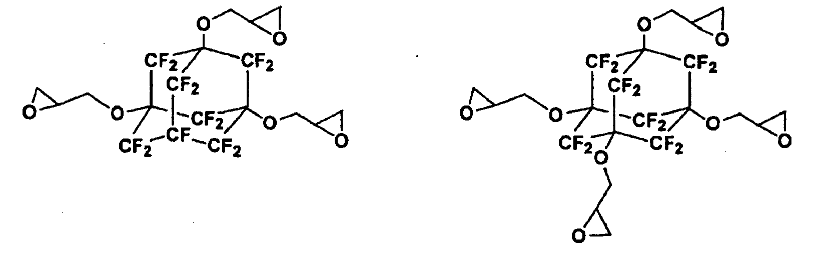

As a method for producing an adamantane compound, a method may be mentioned of reacting, in the presence of an alkali metal hydroxide, a compound (hereinafter referred to as adamantanol) having from one to four of hydrogen atoms bonded to a carbon atom in the adamantane ring substituted by a hydroxyl group and at least six of the remaining hydrogen atoms substituted by a fluorine atom, with epichlorohydrin. The adamantanol is preferably obtained by the method disclosed in WO04/052832.

The adamantanol is preferably the following compound (b), particularly preferably the following compound (b1) : 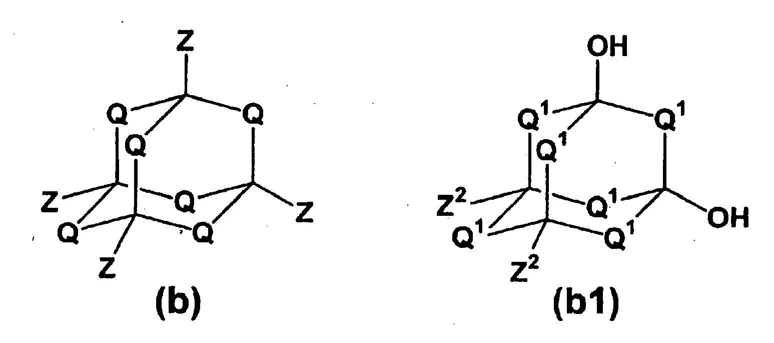 wherein symbols have the following meanings:
Z: each independently a hydrogen atom, a fluorine atom or a hydroxyl group, provided that one to four among four Z's is a hydroxyl group, and
Z²: each independently a hydrogen atom or a fluorine atom.

Z is each independently preferably a fluorine atom or a hydroxyl group. Further, it is more preferred that Z is each independently a fluorine atom or a hydroxyl group, and Q is -CF₂-.

Z² is preferably a fluorine atom. It is more preferred that Z² is a fluorine atom and Q¹ is -CF₂-.

Specific examples of the adamantanol include the following compounds:

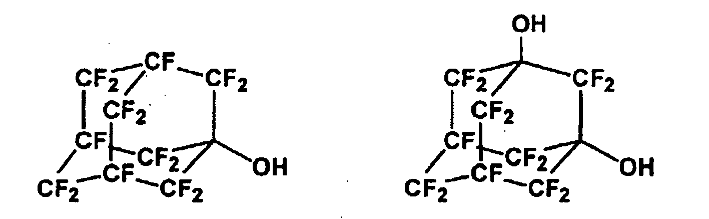

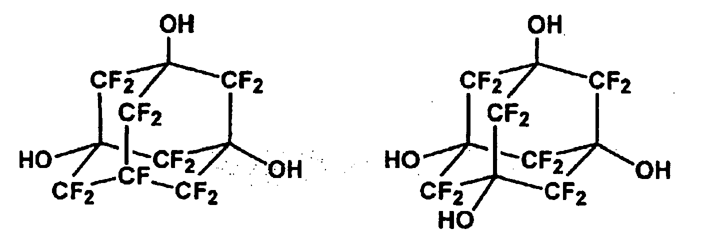

The alkali metal hydroxide is used preferably in 1 to 3 equivalent amounts per equivalent amount of the hydroxyl group in the adamantanol compound. The alkali metal hydroxide is preferably sodium hydroxide or potassium hydroxide.

Epichlorohydrin is used preferably in 2 to 20 equivalent amounts, particularly preferably 5 to 10 equivalent amounts per equivalent amount of the hydroxyl group in the adamantanol compound.

The adamantane compound of the present invention is a reactive fluorinated epoxy compound having a fluorinated alicyclic structure based on an adamantane ring. The adamantane compound of the present invention forms, by curing, a curable fluorinated compound (hereinafter referred to as a cured adamantane product) excellent in light resistance to short wavelength light, transparency and heat resistance.

In the curing, one type of the adamantane compound may be cured or one or more types of the adamantane compounds may be cured. Further, one or more types of the adamantane compounds and another curable compound may be cured. Such another curable compound is preferably the above-described fluoropolymer (curable fluoropolymer) having a group capable of reacting with an epoxy group or an acid anhydride and containing polymerized units based on a fluoroolefin and polymerized units based on a hydrocarbon monomer, and/or acid anhydride. In such a case, they are cured at a proper curing rate to obtain a transparent and colorless cured adamantane product.

In a case where the above curable fluoropolymer is blended, the content of the curable fluoropolymer in the composition is preferably from 5 to 50 mass%, more preferably from 5 to 30 mass%.

The acid anhydride may, for example, be glutaric anhydride, tetrahydrophthalic anhydride, methyltetrahydrophthalic anhydride, hexahydrophthalic anhydride, methylhexahydrophthalic anhydride or methyl nadic acid. The acid anhydride may contain a fluorine atom from the viewpoint of the heat resistance and light resistance of a cured fluorinated product. The acid anhydride containing a fluorine atom may, for example, be perfluoroglutaric anhydride, perfluorosuccinic anhydride or tetrafluorophthalic anhydride.

In a case where the adamantane compound and the acid anhydride are blended, the amount of the acid anhydride is preferably from 0.8 to 1.2 equivalent amounts per equivalent amount of the epoxy group in the adamantane compound.

A cured fluorinated product formed by curing the above adamantane compound is an insoluble and infusible crosslinked polymer and is excellent in adhesive properties, and is excellent in light durability to short wavelength light, transparency, heat resistance and moisture resistance. Therefore, it is useful as a material for an optical article, particularly a material for encapsulating an optical article in a light-transmitting manner. Thus, the present invention also relates to an optical article having the above cured fluorine product. Encapsulation in a light-transmitting manner means encapsulation with both light-transmitting function and encapsulation function. The optical article is preferably a light emitting device or a light receiving device, particularly preferably a light emitting device. Specific examples of the light emitting device include a light emitting diode (LED), a laser light emitting diode (LD) and an electroluminescence (EL) device.

As a method for producing an optical article encapsulated with the cured fluorinated product of the present invention in a light-transmitting manner, a method of applying the adamantane compound to an optical article and then curing the adamantane compound to form a cured fluorinated product, may be mentioned.

### EXAMPLES

Now, the present invention will be described in further detail with reference to Examples, but the present invention is by no means restricted thereto.

In Examples, gas chromatography will be referred to as GC, mass spectrometry as MS, dichloropentafluoropropane as R-225, and polytetrafluoroethylene as PTFE. The GC purity was determined from the peak area ratio in GC analysis. A high-pressure mercury lamp is a high-pressure mercury lamp with an irradiation intensity of 180 mW/cm² at a wavelength of 420 nm, of which light at a wavelength of at most 400 nm was shut off. The light transmittance was determined as a transmittance to light at a wavelength of 400 nm.

### EXAMPLES 1: Example (1) for preparation of adamantane compound

An adamantanol mixture (41.3 g) containing the following compounds (b1F) and (b1H) obtained in the same manner as in Example 2 of WO04/052832 in a molar ratio of 6:4, and epichlorohydrin (74.0 g) were put in a three-necked flask (internal capacity: 300 mL) and stirred to dissolve the mixture. The flask was heated at 80°C and a 50 mass% sodium hydroxide aqueous solution (24 g) was slowly added dropwise, and after completion of dropwise addition, stirring was continued further for 8 hours.

The flask was cooled to 25°C, deionized water (100 mL) and R-225 (50 mL) were added, and the flask was left at rest to obtain a liquid separated into two layers. The lower layer of the solution in the flask was recovered, washed with water three times and dehydrated over magnesium sulfate. Further, the solvent and epichlorohydrin were removed by distillation under reduced pressure to obtain a pale yellow liquid crude product (55 g). A small amount of sodium carbonate was added to the crude product, followed by distillation under reduced pressure to obtain a fraction at 135°C/4 hPa.

The fraction was subjected to GC analysis and as a result, the fraction was confirmed to be an adamantane mixture containing the following compounds (a1F) and (a1H) with a GC purity of 85%, and containing the following compounds (a1F1) and (a1H1) with a GC purity of 15%. The fraction was purified by silica gel column employing a solvent mixture of hexane and ethyl acetate (hexane/ethyl acetate (mass ratio)=1/1) as a developing solvent to isolate compound (a1F) and compound (a1H). The spectrum of the compound (a1F) by EI⁺-MS method was 532, and the spectrum of compound (a1H) was 514. Differential scanning calorimetry of the mixture of compounds (a1F) and (a1H) was carried out and as a result, an endothermic peak accompanying melting was observed at from 40 to 60°C.

¹⁹F-NMR of compound (a1F) (283.7 MHz, solvent: d6-acetone, standard: CFC1₃) δ (ppm): -112.7 (2F), -116.9(8F), -120.7(2F), -220.7(2F)
¹⁹F-NMR of compound (a1H) (283.7 MHz, solvent: d6-acetone, standard: CFCl₃) 5 (ppm): -115.7 to -121.5(8F), -121.2(2F), -220.9(1F), -221.9(1F), -218.7(1F)

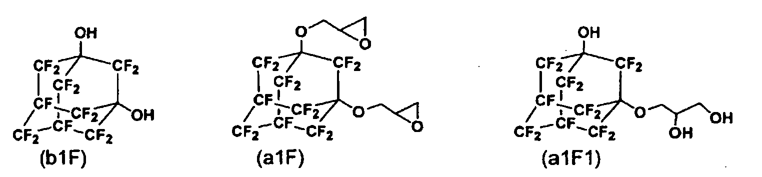

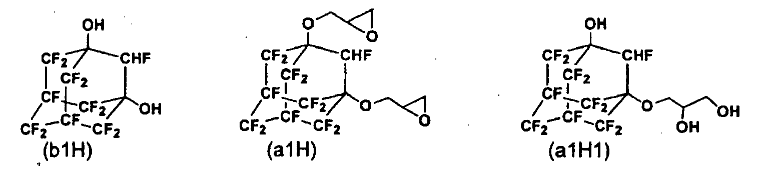

### EXAMPLES 2: Example (2) for preparation of adamantane compound

The same adamantanol mixture (42.5 g) as in Example 1 and epichlorohydrin (92.5 g) were put in a three-necked flask (internal capacity: 300 mL) and stirred to dissolve the mixture. The flask was heated at 60°C, and a 50 mass% sodium hydroxide aqueous solution (24 g) was slowly added dropwise, and after completion of the dropwise addition, stirring was continued further for one hour. Tetrahydrofuran (20 mL) was added to the flask, followed by stirring at 80°C for 10 hours.

The flask was cooled to 25°C, deionized water (100 mL) and R-225 (50 mL) were added, and the flask was left at rest to obtain a liquid separated into two layers. The lower liquid of the solution in the flask was recovered, washed with water three times and dehydrated over magnesium sulfate. Further, the solvent and epichlorohydrin were removed by distillation under reduced pressure to obtain a colorless product (40 g).

### EXAMPLES 3: Example (1) for preparation of cured fluorinated product

100 Parts by mass of the adamantane mixture obtained in Example 1, 65 parts of methylhexahydrophthalic anhydride (hereinafter referred to as MHHPA) and 0.5 part of 1,8-diazabicyclo[5,4,0]undec-7-ene (hereinafter referred to as DBU) were mixed to prepare a liquid composition.

The composition was poured into a mold made of PTFE and cured by heating at 130°C for 5 hours to obtain a transparent and colorless plate-shape cured product (1.5 cm x 1.5 cm x 2 mm in height). The plate-shape cured product had a light transmittance of 91%. Then, the plate-shape cured product was continuously irradiated with a high-pressure mercury lamp at a temperature of at most 100°C for 100 hours. The plate-shape cured product after irradiation had a light transmittance of 87%.

### EXAMPLES 4: Example (2) for preparation of cured fluorinated product

20 Parts of a curable fluoropolymer (weight average molecular weight: 8,200) containing polymerized units based on tetrafluoroethylene, polymerized units based on cyclohexyl vinyl ether and polymerized units based on hydroxybutyl vinyl ether in amounts of 50 mol%, 40 mol% and 10 mol%, respectively, was mixed with and dissolved in 30 parts of R-225. Further, 45 parts of the mixture in Example 1 and 35 parts of MHHPA were mixed and dissolved and then R-225 was distilled off to obtain a liquid composition having a viscosity of about 100 Pa·S at 25°C.

0.5 Part of DBU was added to the composition, and the composition was poured into a mold made of PTFE and cured by heating at 130°C for 5 hours to obtain a transparent and colorless plate-shape cured product (1.5 cm x 1.5 cm x 2 mm in height). The plate-shape cured product had a light transmittance of 90%. The plate-shape cured product after continuous irradiation in the same manner as in Example 3 had a light transmittance of 88%.

### EXAMPLES 5: Example (3) for preparation of cured fluorinated product

30 Parts of R-225 and 25 parts of the same curable fluoropolymer as in Example 4 were mixed and dissolved. Further, 40 parts of the following compound (e) and 35 parts of MHHPA were mixed and dissolved and then R-225 was distilled off to obtain a liquid composition having a viscosity of about 10 Pa·S at 25°C.

0.3 Part of tetra-n-butylphosphonium-o,o-dimethylphosphorodithioate was added to the composition, and the composition was poured into a mold made of PTFE and cured by heating at 130°C for 5 hours to obtain a transparent and colorless plate-shape cured product (1.5 cm x 1.5 cm x 2 mm in height). The plate-shape cured product had a light transmittance of 85%. The plate-shape cured product after continuous irradiation in the same manner as in Example 3 had a light transmittance of 84%.

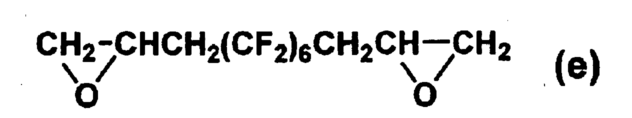

### EXAMPLES 6 (Comparative Example): Example for preparation of cured fluorinated product

A plate-shape cured product was obtained in the same manner as in Example 3 except that hydrogenated bisphenol A glycidyl ether was used instead of the mixture obtained in Example 1. The plate-shape cured product had a light transmittance of 89%, but after continuous irradiation was carried out in the same manner as in Example 3, the light transmittance of the plate-shape cured product decreased to 62%.

### INDUSTRIAL APPLICABILITY

The curable composition and the reactive compound having a novel adamantane ring structure having fluorine atoms and a glycidyl ether group of the present invention provide small volume shrinkage by curing, and are capable of forming a cured fluorinated product excellent in optical properties such as light resistance and transparency. Such a cured fluorinated product is an ' insoluble and infusible crosslinked polymer and is excellent also in adhesive properties and heat resistance and is thereby useful for an optical article.

The entire disclosure of Japanese Patent Application No. 2005-144324 filed on May 17, 2005 including specification, claims and summary is incorporated herein by reference in its entirety.

## Claims

1. A liquid curable composition which comprises a fluoropolymer having a group capable of reacting with an epoxy group or an acid anhydride and containing polymerized units based on a fluoroolefin and polymerized units based on a hydrocarbon monomer, a fluorinated compound having an epoxy group, and an acid anhydride.

2. A cured fluorinated product, formed by curing the curable composition as defined in Claim 1.

3. An optical article having the cured fluorinated product as defined in Claim 2.

4. An adamantane compound having from one to four of hydrogen atoms in the adamantine ring substituted by a glycidyl ether group and at least six of the remaining hydrogen atoms substituted by a fluorine atom.

5. The adamantane compound according to Claim 4, which is represented by the following formula (a): 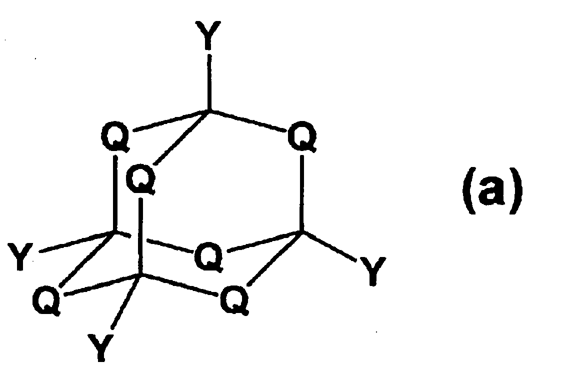 wherein symbols have the following meanings:
Y: each independently a hydrogen atom, a fluorine atom or a glycidyl ether group, provided that one to four among four Y's is a glycidyl ether group, and
Q: each independently -CHF- or -CF₂-.

6. The adamantane compound according to Claim 4 or 5, which is represented by the following formula (a1): 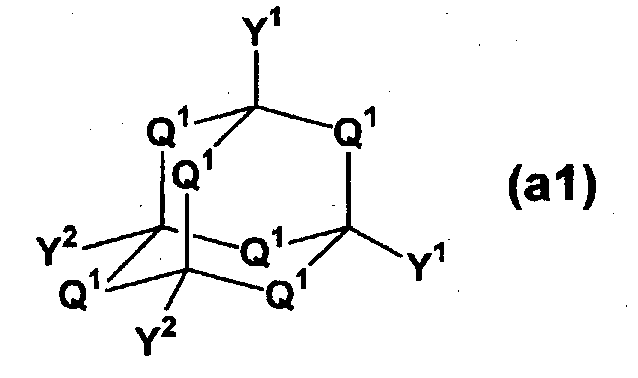 wherein symbols have the following meanings:
Y¹: a glycidyl ether group,
Y²: each independently a hydrogen atom or a fluorine atom, and
Q¹: each independently -CHF- or -CF₂-.

7. A cured fluorinated product formed by curing a liquid curable composition containing the adamantane compound as defined in any one of Claims 4 to 6.

8. A cured fluorinated product formed by curing a liquid curable composition containing the adamantane compound as defined in any one of Claims 4 to 6 and an acid anhydride.

9. A cured fluorinated product formed by curing a liquid curable composition containing the adamantane compound as defined in any one of Claims 4 to 6, a fluoropolymer containing polymerized units based on a fluoroolefin and polymerized units based on a hydrocarbon monomer, and an acid anhydride.

10. An optical article having the cured fluorinated product as defined in any one of Claims 7 to 9.
